(19) Europäisches Patentamt
European Patent Office
Office européen des brevets

(11) Publication number: **0 272 223 B1**

(12) **EUROPEAN PATENT SPECIFICATION**

(45) Date of publication of patent specification: **29.05.91**
(51) Int. Cl.5: **C07D 319/08**, A61K 31/365
(21) Application number: **87830046.6**
(22) Date of filing: **05.02.87**

The file contains technical information submitted after the application was filed and not included in this specification

(54) **4-Oxo-1,3-benzodioxane derivative.**

(43) Date of publication of application:
**22.06.88 Bulletin 88/25**

(45) Publication of the grant of the patent:
**29.05.91 Bulletin 91/22**

(84) Designated Contracting States:
**AT BE CH DE ES FR GB GR IT LI LU NL SE**

(56) References cited:
**EP-A- 0 080 609**
**EP-A- 0 103 320**
**BE-A- 867 831**
**GB-A- 2 018 756**
**US-A- 4 046 887**
**US-A- 4 328 242**

**PATENT ABSTRACTS OF JAPAN, vol. 9, no. 5 (C-260)[1728], 10th January 1985; & JP - A - 59 161 375 (ESUESU SEIYAKU K.K.) 12-09-1984**

**CHEMICAL ABSTRACTS, vol. 100, no. 4, 23rd January 1984, page 446, abstract no. 34473e, Columbus, Ohio, US; A.B. HANSEN et al.: "Chemical feasibility studies concerning potential prodrugs of acetylsalicylic acid", & ACTA CHEM. SCAND., SER. B 1983, B37(4), 351-359**

**CHEMICAL ABSTRACTS, vol. 81, no. 1. 8th July 1974, pages 313, 314, abstract no. 3851m, Columbus, Ohio, US; C. RUECHARDT et al.: "Ambivalent reactivity of O-acetylalicycloyl chloride", & JUSTUS LIEBIGS ANN. CHEM. 1974, (1), 15-23**

**TETRAHEDRON, vol. 39, no. 19, 1983, pages 3151-3157, GB; R. DESTRO et al.: "A paradigm for the structures of cyclic aspirin derivatives. 2-(2-Methoxy-phenoxy)-2-methyl-4-oxo-1,3-benzodioxane"**

(73) Proprietor: **FARMACEUTICI CABER S.p.A.**
**Via Mazzini, 7**
**I-56100 Pisa(IT)**

(72) Inventor: **Torre, Alberto**
**Viale Forlanini 15**
**I-20100 Milano(IT)**

(74) Representative: **Bianchetti, Giuseppe**
**Studio Consulenza Brevettuale Via Rossini, 8**
**I-20122 Milan(IT)**

**EP 0 272 223 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to 2-methyl-2[1-(carboxy-methyl-amino-carbonyl)ethyl] thio-4-oxo-benzodioxane-1.3 of formula I

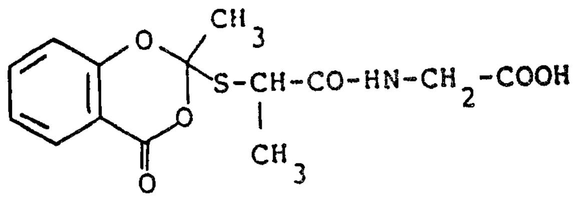

(I)

as well as alkali and alkali-earth metal salts; pharmaceutically acceptable amine salts and basic amino acid salts of compound of formula I.

Lysine, arginine and histidine salts of acid I are particularly preferred.

A cyclic acetylsalicylic acid thioester with α-mercaptopropionylglycine is known from EP-A-0103320.

Compound I and the salts thereof show antiinflammatory, antipiretic and analgesic activity, as well as mucolytic activity. These pharmacologic properties, together with a complete absence of gastro-injuring activity and almost no toxicity (acute, subacute and chronic) make these compounds perfectly tolerated also at the gastric level, and therefore administrable both by oral and rectal route.

Compound I displays its activity particularly at the respiratory district, being therefore particularly suited for the treatment of influenzal diseases and inflammatory conditions of respiratory routes.

The compound according to the invention, which hereinafter will be also defined TF 914, is prepared by reaction between acetylsalicyloylchloride, (2-mercaptopropionyl)glycine and pyridine, in 1:1:1 molar ratios. The reaction is carried out in inert organic solvents, such as dichloromethane.

The 1,3-benzodioxane structure of the compound has been clearly evidenced (Tetrahedron 39/19, 3151-7,-1983).

The following non-limiting example further illustrates the process of the invention.

### EXAMPLE

Acetylsaliciloylchloride was prepared in thionyl chloride at ebollition (about 3 hours), then the excess thionyl chloride was evaporated off and the residue was distilled under vacuum (b.p. 150°/30 mm). Yield 50-70%. The cloride was left to stand at 0-4° C during 18 hours.

N-(2-mercaptopropionyl)glycine (25 g; 0.153 mole) was added to a solution of the above acyl chloride (30 g; 0.15 mole) in chloroform (150 ml).

Anhydrous pyridine (12 ml; 0.15 mole) was added dropwise under vigorous stirring, keeping the temperature of the reaction mixture at 20-25° C.

The mixture was left to stand at room temperature for about 12 hours, then the solution was washed with water (50 ml × 2) and a 5% solution of aqueous sodium hydroxide (50 ml × 2).

The organic phase was dried ($MgSO_4$), filtered and evaporated under reduced pressure, giving a residue which was repeatedly treated with a toluene/ethyl acetate (60:40) mixture, obtaining a solid residue which was dissolved on buchner, washed and dried.

Compound I (14 g; 0.043 mole; 29%) was obtained; m.p. 152-155° C.

I.R. (nujol) in $cm^{-1}$: 3500-2980 (OH e NH stretch.); -C = O ester stretch.; 1720 (C = O acid stretch); 1655 (amidic C = 0) 1130-1120 (C-O-stretch.).

1H-NMR (registered in $CDCl_3$; int. standard TMS.), values of chemical shifts in δ: 1.4 (α , 3H, -CH-$CH_3$); 2.1 (s, 3H;

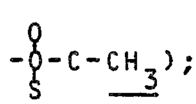

3,6 (9, 1H; CH-$CH_3$); 3.7 (α , 2H; -NH-$CH_2$-); 5.5-6.5 (m, 1H mobile, NH); 6.7-8 (m, 5H; 4H aromatics + -OH mobile).

The pharmacological and toxicological properties of TF 914 were evidenced as hereinafter reported.

TOXITICY

Acute toxicity

Toxicity by single administrations of TF 914 was studied in the mouse and the rat, by oral and intraperitoneal route.

$LD_{50}$ values were calculated according to the method of Litchfield and Wilcoxon (J. Pharm. Exp. Therap., 1949, 96, 99).

The obtained results, which are summarized in table 1, evidenced that TF 914 has no toxicity.

TABLE 1 - Acute toxicity of TF 914

| SPECIES | ADMINISTRATION ROUTE | $LD_{50}$ mg/kg |
|---|---|---|
| Mouse | os | > 3.000 |
| | i.p. | > 3.000 |
| Rat | os | > 3.000 |

PHARMACOLOGY

1. Antiinflammatory activity

a. Carrageenin oedema

Antiinflammatory activity of TF 914 was evaluated by means of the carrageenin oedema test in the rat, according to the method of Winter, Rissly, Ness (Proc. Soc. Exp. Biol. Med., 1962, 111, 544), in comparison with acetylsalicylic acid at equimolecular doses.

The compound under test and acetylsalicylic acid, as a comparison, were administered by the oral route 30 minutes before subplantar injection of carrageenin. Plethysmographic evaluations were carried out every hour during 7 hours after the induction of the oedema.

The obtained results, which are shown in table 2, evidence the marked antiinflammatory activity of TF 914, said activity being higher than that of acetylsalicylic acid in equimolecular doses, under the experimental conditions used.

TABLE 2 - Antiinflammatory activity - Carrageenin oedema in the rat.

| TREATMENT | DOSES mg/kg p.o. | N. ANIMALS | PAW/VOLUME ml/10 - $\bar{M} \pm ES$ Basal | 2h | 4h | 6h | 8h | 24h | AREAS Δ % $M \pm ES$ | inhib. vs. contr. |
|---|---|---|---|---|---|---|---|---|---|---|
| Control | - | 10 | 40,0 ± 0,7 | 72,0 ± 2,9 | 63,2 ± 1,9 | 59,7 ± 1,8 | 55,4 ± 2,4 | 47,8 ± 1,4 | 256,4 ± 19,8 | - |
| ASA * | 100 | 10 | 43,1 ± 1,5 | 58,3 ± 2,5 | 66,0 ± 3,0 | 59,3 ± 1,7 | 52,8 ± 2,6 | 45,3 ± 1,5 | 161,5 ± 20,9 | 37,0 |
| TF 914 | 180 | 10 | 42,8 ± 0,7 | 53,3 ± 1,6 | 60,4 ± 2,0 | 61,8 ± 2,7 | 54,5 ± 2,8 | 45,1 ± 1,5 | 145,4 ± 20,4 | 43,3 |

* Acetylsalicylic acid

b. Carrageenin pleuritis

The antiinflammatory activity was tested by the carrageenin induced pleuritis test in the rat, according

to the method of Vinegar R. et al. (Proc. Soc. Exp. Biol. Mod., 1973, 143, 3).

TF 914 and phenylbutazone as a control were administered orally in equiponderal doses, 1 hour before the carrageenin injection in the pleuric cavity.

Five hours later the animals were killed, and volume of the pleuric exudate and total number of leukocytes therein were evaluated.

The obtained results proved that TF 914 exherts a 27.2% protection on the exudate volume and a 19.5% on the leukocytes number, 5 hours after the treatment.

This evidence appears to be particularly important, for both the measurement of the activity per se, and the comparison with the activity showed by a well-established comparison drug.

The results of study are reported in table 3.

TABLE 3 - Carrageenin pleuritis

| TREATMENT | DOSES mg/kg p.o. | WEIGHT g | TIME (h) | EXUDATE VOLUME ml | EXUDATE VOLUME % vs.contr. | N. LEUKOCYTES ml | N. LEUKOCYTES % inhib. vs. contr. |
|---|---|---|---|---|---|---|---|
| CARRAGEENIN | — | 281,0 ± 2,44 | 5 | 2,46 ±0,25 | — | 13,46 ±1,25 | — |
| PHENYLBUTAZONE | 100 | 277,0 ± 7,34 | 5 | 1,75 ±2,25 | 28,9 | 8,52 ±1,27 | 36,7 |
| TF 914 | 100 | 268,0 ± 3,74 | 5 | 1,79 ±0,25 | 27,2 | 10,84 ±1,32 | 19,5 |

2. Analgesic activity

The analgesic activity was studied by the phenylquinone writhing test in the mouse according to the

method of Siegmund E. et al. (Proc. Soc. Exp. Biol. Med., 1957, 95, 729). TF 914 was orally administered, in comparison with acetylsalicylic acid, in equimolecular doses. The two drugs were administered 30 min. before intraperitoneal injection of phenylquinone.

The obtained results are reported in table 4, which evidences the high analgesic activity of TF 914, which activity is comparable to that of acetylsalicylic acid in equimolecular doses, under the used experimental conditions.

TABLE 4 - Analgesic activity - Phenylquinone writhing in the mouse.

| Treatment | Dose mg/kg os | Mean number of writhings | % inhib. vs. contr. | N. animals showing writhing |
|---|---|---|---|---|
| Controls | - | 12.8 ± 2.6 | - | 10/10 |
| Acetylsalicylic acid | 100 | 1.4 ± 0.9 | 89.06 | 3/10 |
| TF 914 | 180 | 1.8 ± 1.1 | 85.93 | 3/10 |

3. Bronchosecretolytic activity

The bronchosecretolytic activity was evaluated in the male Wistar rat by oral administration of TF 914 according to the method of Mawatari (Mawatari H. Kagoshima Daigaku Igaku-Zasshi- 27, 561, 1976), in comparison with Thiola®, in equimolecular doses. The results are listed in table 5.

TABLE 5 - Bronchosecretolytic activity in the rat after oral administration of TF 914 and Thiola[R] at equimolecular doses.

| Treatment | Dose mg/kg os | N. of animals | % increase of FlNa in comparison with controls |
|---|---|---|---|
| TF 914 | 360 | 10 | 35.6 |
| Thiola® | 200 | 10 | 33.4 |

The above reported data prove that the compounds under test both have a marked bronchosecretolytic activity, which is substantially superimposable, after administration of equimolecular doses of the compounds, under the used experimental conditions.

4. Gastro-injuring action

The action of equimolecular doses of TF 914 and acetylsalicylic acid on gastric mucosa was evaluated by administering the products by the oral route to rats fasted for 18 hours at least. The examination of the mucosa of the animals 6 hours after the treatment, evidenced that TF 914 substantially has no gastro-injuring action, while acetylsalicylic acid showed marked gastro-injuring effects, as clearly shown by the results reported in table 6.

TABLE 6 - Gastro-injuring action of TF 914

| Treatment | Dose mg/kg/os | Ulcer size (mm) $\bar{x} \pm es$ |
|---|---|---|
| Controls | - | 0.0 ± 0.0 |
| ASA | 100 | 3.6 ± 1.34 |
| TF 914 | 180 | 0.4 ± 0.20 |

PHARMACOKINETICS

The pharmakinetics studies on TF 914 were carried out in the rat, in comparison with acetylsalicylic acid and Thiola®, after oral administration of equimolecular doses of the three compounds.

Measurements were performed by HPLC.

From the obtained results, TF 914 proved to have a better bioavailability and to remain in blood stream for longer periods than the two single components.

Moreover, a surprisingly marked concentration was evidenced in the lung, said concentration being substantially higher than the one attained by separately administering the two components.

The above mentioned characteristics make TF 914 particularly useful for the treatment of obstructive and inflammatory conditions of the respiratory system.

The present invention also relates to all the industrial aspects of the invention connected to therapeutic use of TF 914.

Another object of the invention is provided by pharmaceutical compositions containing pre-set therapeutically effective amounts of TF 914, in admixture with pharmaceutically acceptable carriers or diluents. Non-limiting examples of said pharmaceutical compositions are capsules, tablets, sugar-coated pills, syrups, suppositories, injection vials.

## Claims

1. 2-methyl-2[1-(carboxy-methyl-amino-carbonyl),ethyl] thio-4-oxo-benzodioxane-1,3 of formula I

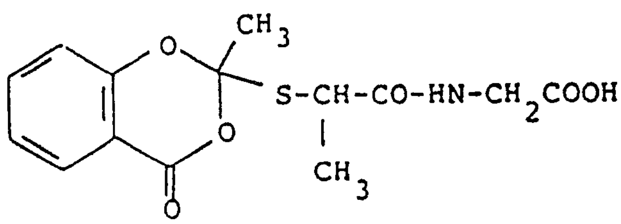

(I)

as well as alkali and alkali-earth metal salts; pharmaceutically acceptable amine salts and basic amino acid salts of compound of formula I.

2. Salts of compound I with alkali and alkali-earth metals and with basic aminoacids such as lysine, arginine, histidine.

3. A process for the preparation of the compound as claimed in claim 1, which process comprises reacting acetylsalicyloyl chloride with N-(2-mercaptopropionyl) glycine and pyridine, in an inert organic solvent.

4. A process according to claim 3, wherein chloroform is used as the organic solvent.

5. Pharmaceutical compositions having antiinflammatory, antipiretic and mucolytic activities, containing as the active ingredient therapeutically effective amounts of at least one compound as claimed in claims 1 or 2.

6. Pharmaceutical compositions according to claim 5, for oral, parenteral or rectal administration, in form of tablets, capsules, sugar-coated pills, syrups, suppositories, injectable solutions.

7. Compounds according to claims 1 or 2 for use as medicaments.

## Revendications

1. 2-méthyl-2 1-(carboxy-méthyl-amino-carbonyl) éthyl thio-4-oxo-1,3-benzodioxane de la formule I

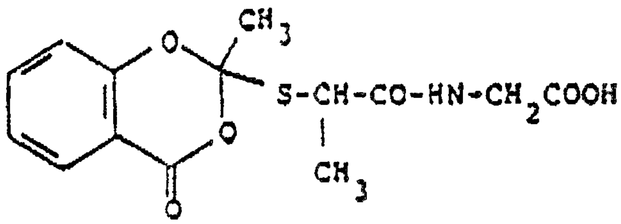

(I)

ainsi que les sels des métaux alcalins et alcalinoterreux, les sels aminés pharmaceutiquement acceptables et les sels des acides aminés basiques, du composé de formule I.

2. Sels du composé I avec des métaux alcalins et alcalino-terreux, et avec des acides aminés basiques tels que la lysine, l'arginine et l'histidine.

3. Procédé pour la préparation du composé selon la revendication 1, lequel procédé comprend la réaction du chlorure d'acétylsalicyloyle avec le N-(2-mercaptoproprionyl) glycine et la pyridine, dans un solvent organique inerte.

4. Procédé selon la revendication 3, dans lequel le solvent organique est le chloroforme.

5. Compositions pharmaceutiques ayant des activités anti-inflammatoire, anti-pyrétique et mucolytique, et contenant comme principe actif une quantité efficace thérapeutiquement d'au moins l'un des composés selon l'une des revendications 1 ou 2.

6. Compositions pharmaceutiques selon la revendication 5, pour administration orale, parentérale ou rectale, sous forme de comprimés, d'ampoules, de dragées enrobées de sucre, de sirops, de suppositoires, et de solutions injectibles.

7. Composés selon l'une des revendications 1 ou 2 pour utilisation comme médicament.

## Ansprüche

1. 2-Methyl-2[1-(karboxy-methyl-amino-karbonyl)ethyl] thio-4-oxo-benzodioxan-1,3 der Formel I

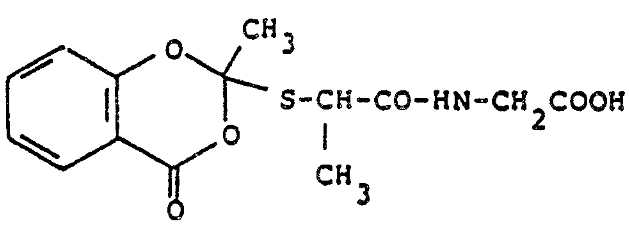

(I)

sowie Alkali- und Erdalkalimetallsalze; pharmazeutisch zulässige Aminsalze und basische Aminosäuresalze der Verbindung der Formel I.

2. Salze der Verbindung I mit Alkali- und Erdalkalimetallen und mit basischen Aminosäuren wie Lysin, Arginin, Histidin.

3. Verfahren zur Herstellung einer Verbindung nach Anspruch 1,
wobei das Verfahren ein Reagieren von Azetylsalizyloylchlorid mit N-(2-merkaptopropionyl)glycin und Pyridin in einem inerten organischen Lösungsmittel aufweist.

4. Verfahren nach Anspruch 3,
wobei Chloroform als organisches Lösungsmittel verwendet wird.

5. Pharmazeutische Zusammensetzungen mit entzündungshemmenden, fiebersenkenden und schleimlösenden Eigenschaften, die als aktiven Bestandteil therapeutisch wirksame Mengen von mindestens einer Verbindung nach Anspruch 1 oder 2 enthalten.

6. Pharmazeutische Zusammensetzungen nach Anspruch 5 zur oralen, parenteralen oder rektalen Verabreichung in Form von Tabletten, Kapseln, zuckerüberzogenen Pillen, Sirups, Zäpfchen, injizierbaren Lösungen.

7. Verbindungen nach Anspruch 1 oder 2 zur Verwendung als Arzneimittel.